Europäisches Patentamt

(19) European Patent Office

Office européen des brevets

(11) EP 0 257 922 B1

(12) **EUROPEAN PATENT SPECIFICATION**

(45) Date of publication and mention
of the grant of the patent:
**31.01.1996  Bulletin 1996/05**

(51) Int. Cl.$^6$: **A61B 6/03**,  A61B 5/04

(21) Application number: **87307135.1**

(22) Date of filing: **12.08.1987**

(54) **System for dynamic scan at cyclic displacement**

Anordnung zur dynamischen Abtastung während periodischer Verstellung

Système de balayage dynamique lors de déplacements périodiques

(84) Designated Contracting States:
**DE**

(30) Priority: **12.08.1986 JP 189173/86**

(43) Date of publication of application:
**02.03.1988  Bulletin 1988/09**

(73) Proprietor: **KABUSHIKI KAISHA TOSHIBA**
**Kawasaki-shi,  Kanagawa-ken 210,  Tokyo (JP)**

(72) Inventors:
• **Mori, Issei**
**Minato-ku  Tokyo 105 (JP)**
• **Yoshida, Tadatoki**
**Minato-ku  Tokyo 105 (JP)**

(74) Representative: **BATCHELLOR, KIRK & CO.**
**London EC1R 0DS (GB)**

(56) References cited:
**EP-A- 0 018 872**          **EP-A- 0 041 752**
**US-A- 4 182 311**

## Description

This invention relates generally to medical diagnostic apparatus and more specifically relates to X-ray scanning apparatus associated with computed tomography. Examples of such apparatus include X-ray computed tomography devices and magnetic resonance imaging device (MRI) which are medical diagnostic devices for producing images of sectional planes of a patient.

The invention relates particularly to applications in which parts of the body move rhythmically for example because of the action of the heart or because of respiration. It is known, e.g. from EP-A-41 752, to use dynamic scanning to follow the change of the image by injecting contrast medium into the body and following its flow with the elapse of time.

In view of the movement of the heart, in the case of a device which is slow in scanning, synchronised scanning is carried out to avoid artifacts and blurring of images and so as to obtain useful images, this principle is used in US-A-4 182 311.

In this method, the scanning process is repeated 10 times for example, electrocardiographic waves are recorded each time, and the projection data obtained at the required timing with a specific phase condition (having a certain narrow width) of the electrocardiographic waves are collected together from the projection data groups obtained from a plurality of scannings to form an image at the specific phase condition.

Thus a chart of the images obtained as above, is made from the projection data acquired over 10 scanning periods. If 5 seconds is assumed to be the time required for one complete scan the time width of the chart is 50 seconds and it is thus impossible to follow rapid circulation of the blood. As the scan time of existing X-ray CT devices is normally several seconds, a diagnostically useful image cannot be obtained without synchronised scanning.

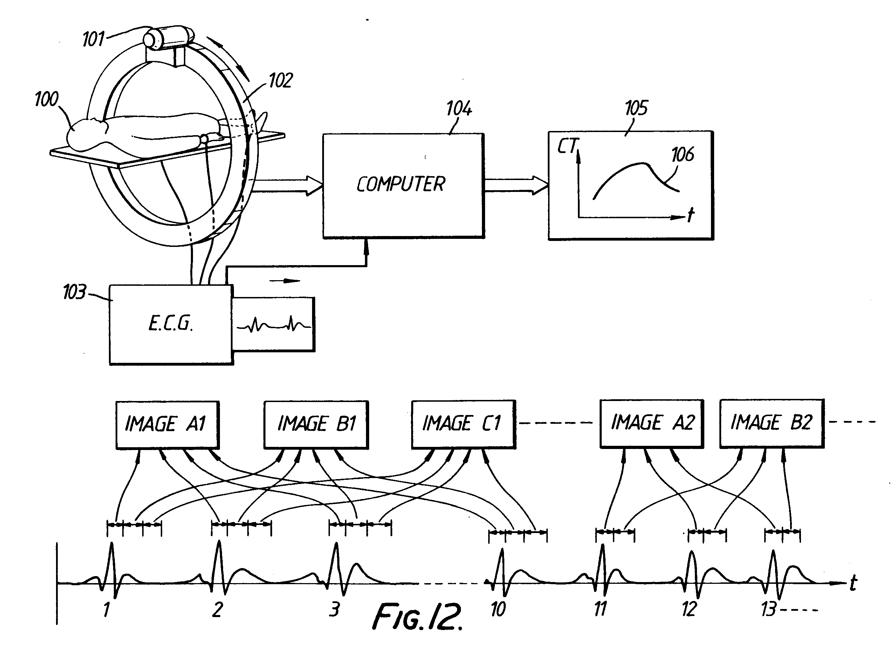

Figure 12 shows how an image is reconstructed by collecting together the projection data from the same phase of each scan.

X-rays are emitted toward a patient 100 from an X-ray source 101 and X-ray absorption factor of the patient 100 is detected by a ring-like X-ray detector assembly 102. Simultaneously an electrocardiogram of the patient 100 is read by an ECG 103.

From the output of the ECG 103 and the detected data from the detection 102, a reconstructed image is produced by a computer 104 and a dynamic curve 106 is displayed on a display 105. The dynamic curve 106 shows the change in CT number, in an optional portion of the reconstructed image.

According to Figure 12, an image A1 consists of R-waves from heart beats 1 to 10, and an image B1 consists of 10 S-T waves. Image A2 is a reconstructed image of R-waves from heart beats 11 to 20.

When images are reconstructed by selecting the detected data in this way, even sectional regions which are vibrating synchronously can be displayed as a stationary image, allowing proper diagnosis.

However, when observing change in CT number in an optional position by using such sectional image, since the images include data over a long period from heart beats 1 to 10, the error becomes large. In Figure 12 the error corresponds to the value of 10 times one heart beat and if one second is required for one heart beat, the total time error is 10 seconds which is very large.

Here, above mentioned error means deterioration of temporal resolution. The images A1, A2, ---,B1, B2, --- and so on are reconstructed from projection data collected over a period of 10 heart beats. A dynamic curve can be drawn from images of A1, A2 and so on. Since these are images of same cardiac phase, spatial blurring can be avoided and this is the big virtue of this method. However, the problem is that the dynamic curve obtained in this way s very poor in temporal resolution due to 10 seconds time duration and cannot be used to observe rapid change of CT number of myocardial muscle by contrast media.

It is also possible to obtain dynamic curve 106 in another way without use of EC gram. For instance, images are reconstructed using projection data collected during each one rotation. From these data, dynamic curve 106 can be drawn without deterioration of with temporal resolution corresponding to the time for one rotation. However, the images are not synchronised with heart beat. Some images are dominated by systolic phase and some are dominated by diastolic phase. Consequently, the locations of the myocardial muscle are different image by image. Therefore it is difficult to set ROI in the images accurately, and the resultant dynamic curve 106 will contain fluctuations due to heart beat. Also there is no guarantee that the obtained dynamic curve shows CT number change of myocardial muscle, since it may be contaminated by portion other than myocardinal muscle.

Accordingly the present invention provides a computer tomographic system for dynamic scanning of an object which is subject to cyclic displacement, as defined in claim 1.

Some embodiments of the invention will now be described by way of example with reference to the accompanying drawings in which:

Figure 1a is a schematic block diagram illustrating the dynamic scan system according to the invention;
Figure 1b is a schematic diagram illustrating the relation between the scan images and the patient's ECG shown in Figure 1a;
Figure 2 is a schematic diagram illustrating each projection data relating to rotation of the source;
Figure 3 is a schematic table illustrating projection number and phase of wave "R" according to the invention;
Figure 4 is a schematic block diagram illustrating the dialogue machine and computer;

Figure 5 is a schematic table illustrating slice numbers, respective projection numbers and phase;

Figure 6 is a schematic diagram illustrating each scan image;

Figure 7 is a schematic diagram illustrating the method of positioning of ROI with the display and the dialogue machine;

Figure 8 is a schematic diagram illustrating a dynamic curve according to the invention;

Figure 9 is a schematic block diagram illustrating a computer according to the invention;

Figure 10 is a schematic table of relation between slice numbers and projection numbers in case of phase of wave "R" to "R" interval;

Figure 11a is a schematic block diagram illustrating the dynamic scan system according to the invention;

Figure 11b is a schematic diagram illustrating the relationship between the scan image and the patient's ECG shown in Figure 11a;

Figure 12 is a diagram illustrating the dynamic scan system.

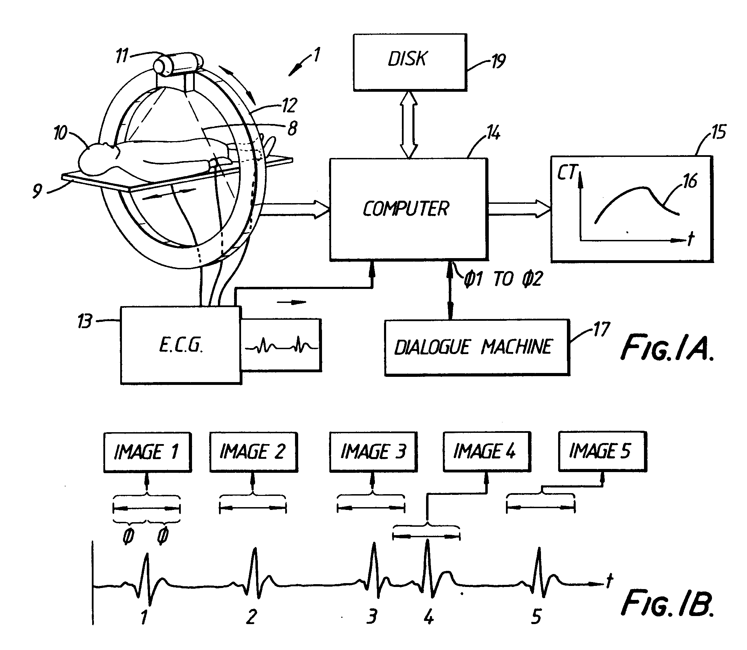

Figure 1 of the drawings illustrates a system for dynamic scanning an object at a cyclic displacement, having a X-ray scanning apparatus for example 4th-generation computed tomography apparatus.

In Figure 1a herein an external perspective view appears, the view being somewhat simplified in nature, and setting forth scanning apparatus 1 of a type suitable for use with the invention. This view should be considered simultaneously with the block diagram and electrocardiograph. This figures discloses the general prior art arrangement of the devices illustrated therein, and serves primarily to establish the background for illustrating the present invention.

Apparatus 1a is a 4th-generation CT, and comprises generally a couch 9 being moved along an axis so as to enable movement of a patient 10 into the apparatus, a radiation source 11 comprising an X-ray source capable of projecting an X-ray pattern in the form of a fan beam 8 rotating continuously around the patient 10, and detector means 12 in a ring around the same axis as the rotation of the X-ray source 11 for detecting radiation from the source 11 via the patient 10.

The signals from the detector means 12 corresponding to the intensity of radiation are suitably converted so as to be sent to a computer 14, and then are reconstructed there so as to enable diagnosis as CT number, CT tomogram, Dynamic curve 16 and so forth.

The basic system and method of use of CT apparatus of this kind is disclosed in detail for example in U.S. Patent No. 4,206,362 and U.S. Patent No. 4,075,492.

A cardiac computed tomograph comprises generally an electrocardiograph 13 together with CT scanner 1. ECG 13 detects electrical movement of the patient's heart, records it as an electrocardiogram and provides a corresponding output to the computer 14, and also sends to the computer 14, the communication data between an operator and the dialogue machine 17.

Figure 1b discloses the relationship between the electrocardiogram of the patient 10 and the portions of the used projection data required to reconstruct each image in the first embodiment.

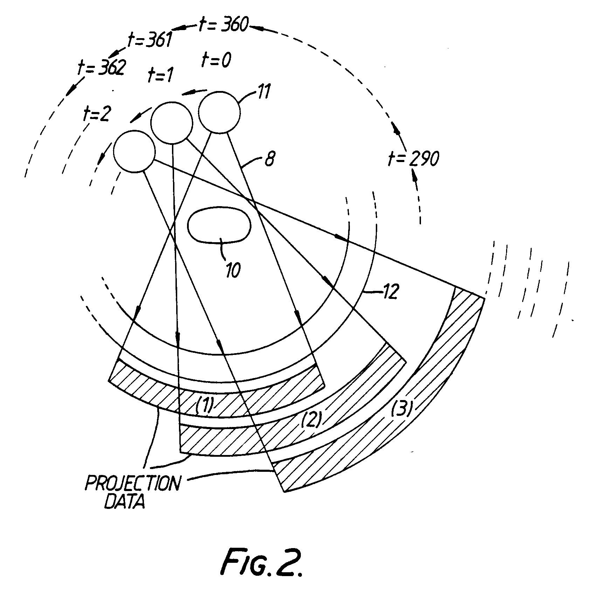

Figure 2 discloses projection data (1), (2), (3)--- at each section t=0,1,2--- of the X-ray source 11. At the section t=0, projection data are detected by the part of the detector means 12 located opposite the source 11. The X-ray source 11 rotates continuously around the patient 10 projecting an X-ray pattern in the form of fan beam 8. Rotation of the source 11 takes for example 0.47 sec from t=0 to t=360 in a counterclockwise direction in Figure 2. Thereby the positions of the source 11, at which projection data (1), (361), (721)--- are detected are the same.

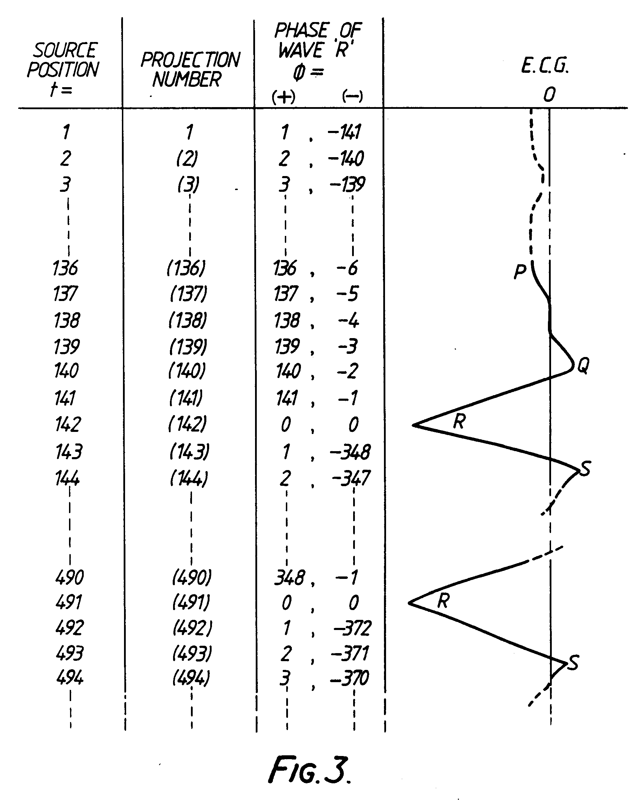

In Figure 3, a source position t refers to the position of the X-ray source 11 while it is rotating by constant-velocity driving means as in Figure 2. Thus t=0 and t=360 are corresponding positions. Each projection number corresponds to source position t.

The EC Gram of the patient 10 is sent from ECG 13 to the computer 14. If the ZERO phase of wave "R" is defined as the peak of wave "R", a change in phase of ECG corresponds to each projection number. The projection data, projection numbers and phase $\phi$ of the ECG are sent to disk 19 and stored there.

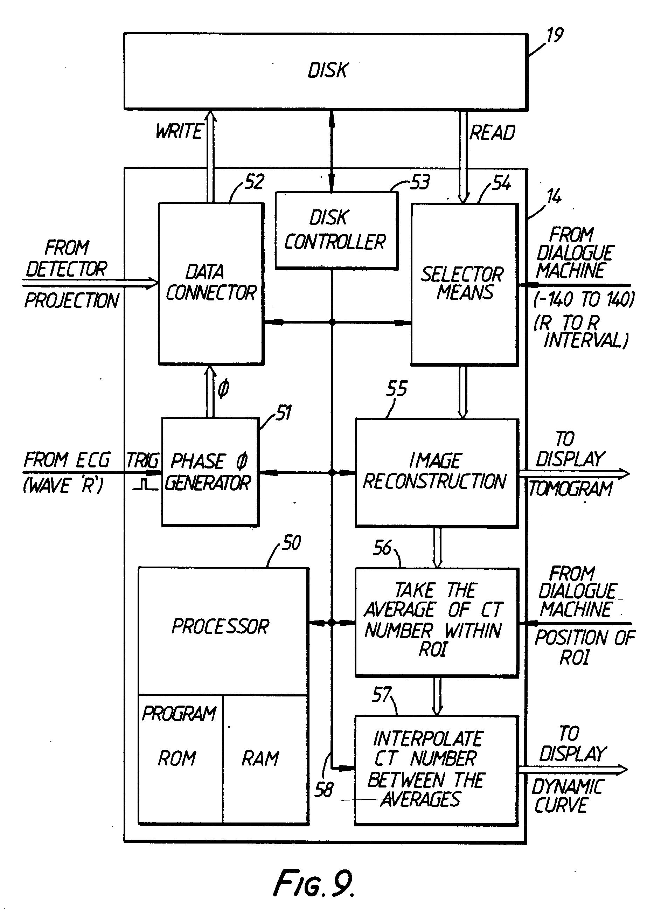

Figure 9 shows the configuration of computer 14. Phase generator 51 provides to data connector 52 a phase angle $\phi$ for the electrocardiogram. The base position of phase $\phi$ is triggered from the top of wave "R" from ECG because it is the most distinguishable feature of the EC Gram.

Phase $\phi$ and projection data and number are transmitted by data connector 52 to disk 19 and stored.

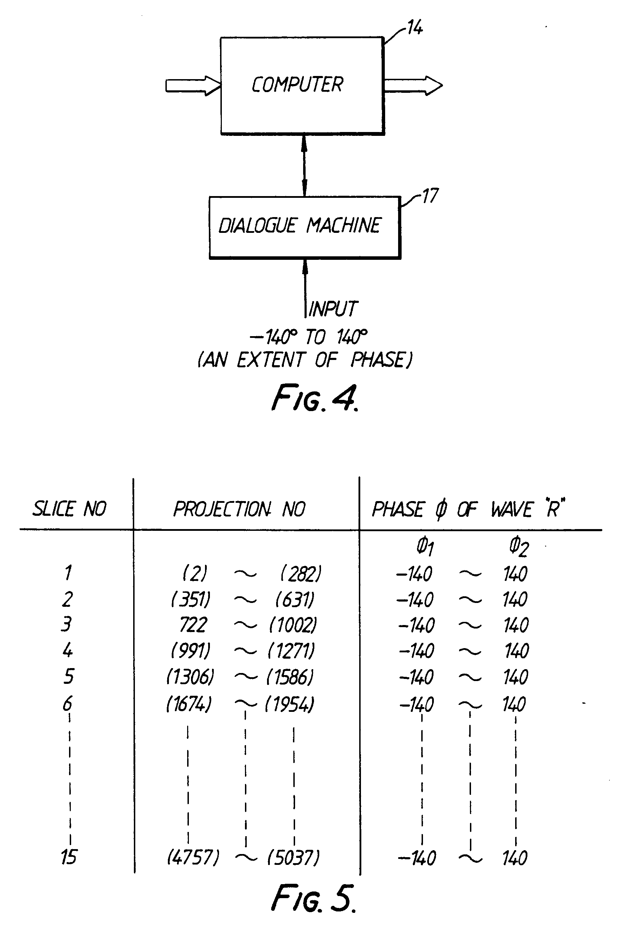

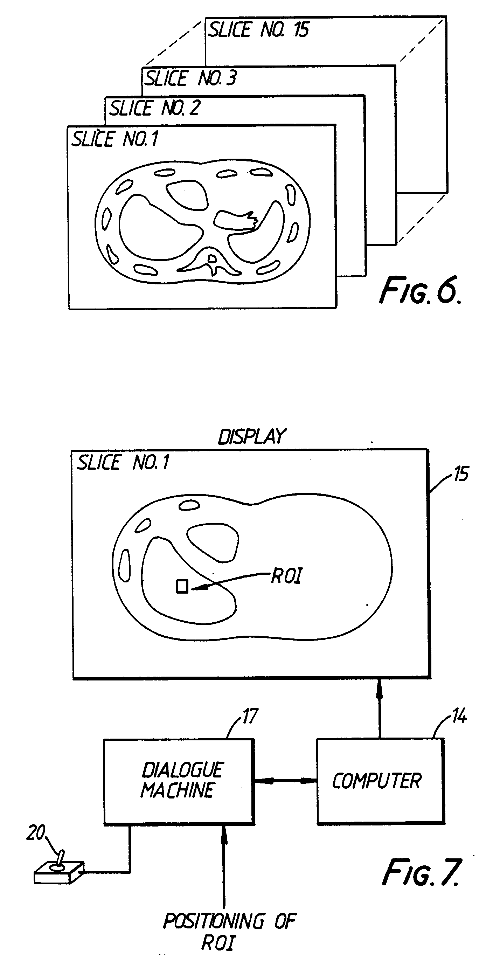

Figure 4 shows the circumstances for inputting phase $\phi$ for example -140° to +140° via the dialogue machine 17 between the operator and the computer 14. The phase signals are sent to the selector means 54. The selector means 54 reads phase $\phi$ and projection data and number from the disk 19 based on data $\phi$ from the dialogue machine 17, and then projection data corresponding to phase $\phi$ designated by the operator are sent to an image reconstruction 55, and reconstructed there as a scan image of the patient. The scan image is shown in Figure 6. Figure 5 shows consecutive numbers of projection corresponding to each slice in the case where the phase $\phi$ of wave "R" is in the range from -140° to +140°. Projection numbers (2), (3), ---, (282) are used to reconstruct slice number 1 in Figure 5. These projections for example (2) to (282) are decided on the basis of the table and phase $\phi$ for example -140° to +140° in Figure 3. Similarly the projections of other slices are decided basing on it.

Here, in the case that the "R" to "R" interval is over $\phi_2-\phi_1=280$ (slice No.3 in Figure 5), the reconstruction of slices No.3 and No.4 make use of the same projection data (991), (992),---, (1002).

The operator positions the ROI (region of interest) via the dialogue machine 17 with a joystick 20 or a trackerball, on the image of the patient 10.

The image reconstructed by reconstruction means 55 is provided to means 56 for taking the average of CT number within ROI and to the display 15 shown in Figure 7 and expressed on the display 16.

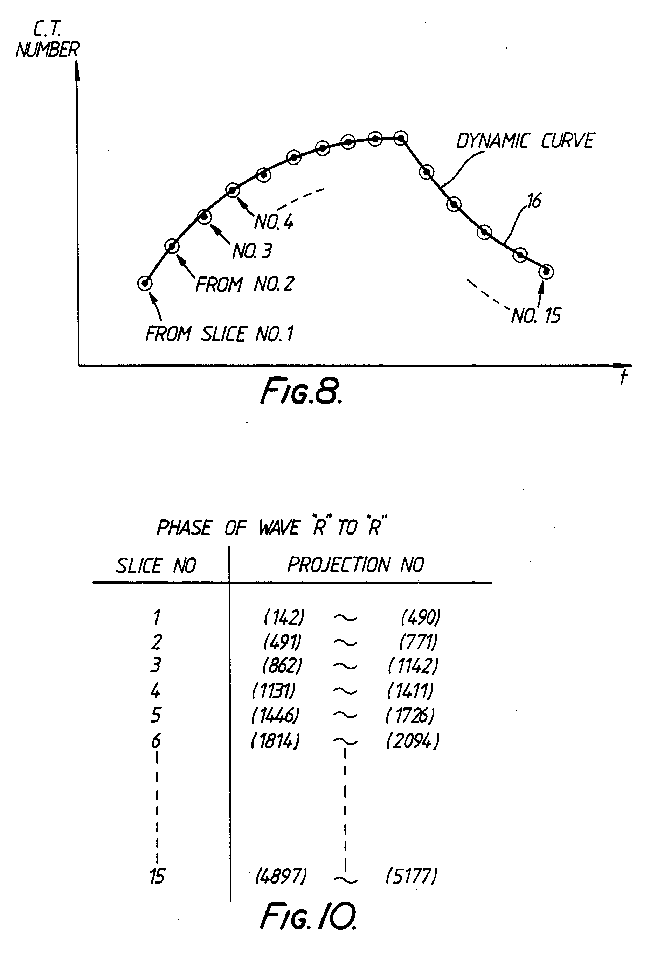

There is generally an area in ROI. Accordingly the average of a number of CT values within ROI at 56 are used by the computer 14. At 57, the CT number is interpolated between the CT numbers provided at 56. The chain of CT numbers interpolated at 57 is sent to the display 15, and expressed as a dynamic curve 16 in Figure 8.

The dynamic curve 16 shows the variation of CT numbers over the ROI chosen by the operator.

As the dynamic curve 16 in this invention offers a chain of accurate CT numbers, the doctor can diagnose the patient 10 more accurately.

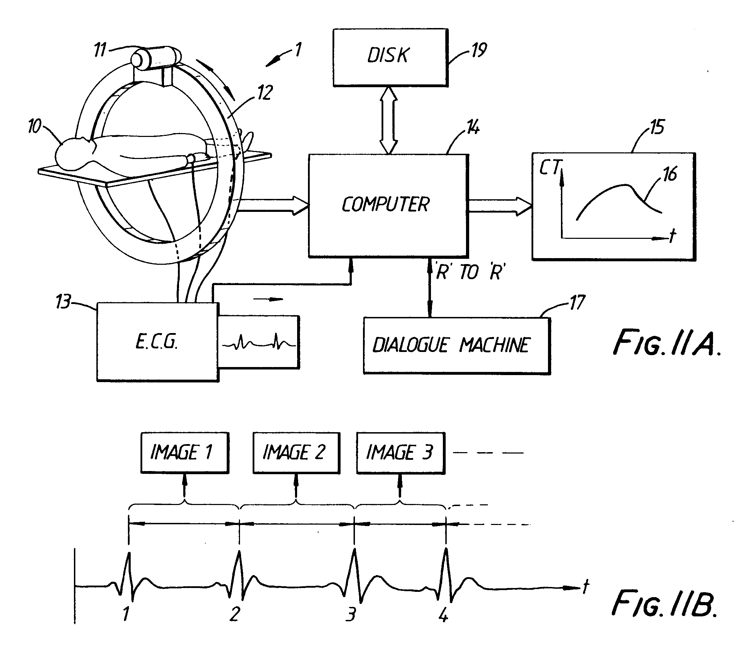

Figures 11a and 11b disclose the second embodiment. In this second embodiment $\phi 1$ and $\phi 2$ in the first embodiment are not used. Instead wave "R" and next "R" of the ECG are used. The relationship between slice number and projection numbers is shown in Figure 10. The relationship between source position, projection number and heart beat is shown again by Figure 3. In Figure 11b, the image 1 is reconstructed by the projection data detected at an interval between wave "R" No. 1 to "R" No. 2.

The computer 14 reads the projection data which were obtained during the time period beginning from the time at which phase $\phi$ is zero (namely the timing at R wave) to the time at which phase $\phi$ is next zero (namely the timing at next R wave). In this manner, image 1, image 2, image 3 and so on are reconstructed. From these images, dynamic curve 16 can be generated for a ROI set in similar manner as the first embodiment.

The first and second embodiments both operate by reconstructing, time-sequential images from which dynamic curve 16 is drawn. For the image, centre phase of cardiac beat can be defined. The centre phase of the first embodiment is $(\phi_1 + \phi_2)/2$, centre phase of the second embodiment is mid-point of R to R. All images used to draw dynamic curve have identical central phase. Therefore, despite the heart beat, the object such as myocardial muscle stays at the same position in the images. ROI for dynamic curve can be set accurately, and the concern of contamination by other part than the intended part (such as myocardial muscle) is substantially reduced. Thus, the obtained dynamic curve is useful and reliable to observe rapid change of CT number or ROI. Thus an image can be reconstructed from the projection data having a specified width and centred by the specific centre phase and also in the same manner as the second embodiment, as each image consists of the projection data having its centre in the specific phase there is no phase displacement between images, and even a periodically moving portion such as the heart, the object can be made to appear stationary in a series of images changing with the elapse of time.

In both of first and second embodiments from the value $\phi_1 - \phi_2$ or the time duration between R to R and from the valuation to the scanning time (required for one rotation of the table), the projection data for reconstructing a plane of image, increases or decreases compared to that required by one rotation of the table. This problem can be solved by providing either one of the following procedures:

(1) When the projection data includes more quantities than those corresponding to one rotation of the table:

1 In the data from the angle of rotation $\alpha$ to $2\pi+\beta$ ($\alpha < \beta$), the projection data corresponding to $2\pi$ centred by $\pi+(\alpha+\beta)$ may be used.
2 Regarding the duplicated portion as below, the weighted means of both the values may be used.

$$P(\phi) = P(\phi) \text{ -------------------- } \alpha+\beta < 2\pi+\alpha$$

$$P(\phi) \times K + P(\phi + 2\pi).(1-K) \text{ ---} \alpha < \phi < \beta$$

Where, the above coefficient K changes monotonically from 0 to 1.

(2) When the projection data includes fewer quantities than corresponding to one rotation of the table:

When the projection data has more quantities than that of the 180° + X-ray fan beam scanning angle, reconstruction of the CT image is possible. For example, the article by D. Parker "Optimal Short Scan Convolution Reconstruction", Medical Physics 9(2), March/April/1982 describes that correct image construction is possible. Therefore, for example, the operation may be executed either by extracting the required portion (1/2 rotation angle + X-ray fan beam angle) of projection data centred by $(\phi_1 + \phi_2)/2$. Or, in accordance with the above mentioned article, the main points of which is when both projection data are collected together from 180° opposite sides the image is reconstructed by weighting the data so that their total sum becomes 1 and by usual convolution/back projection, so the same weighting method may be applied to data exceeding 1/2 rotation angle + X-ray fan beam angle.

The periodic variation may be caused by a movement other than the heart described in the embodiment but the same principle can be applied to the varying portion, for example in the case of respiration. In this case, phase synchronisation can be achieved by recording the respiration waveform.

Moreover, even in devices in which continuous scanning is not performed, for example, in the case of electron beam scanning described in "Radiology of Skull and Brain", Vol 15 PP4366 to PP4369, high speed scanning is repeated and the images are produced by the projection data obtained from average scan data for acquiring high quality images with less noise and in that case, making an image by projection data having a specified width centred by the specific phase of the varying period is very useful (since by the repeated scannings the scan time is substantially lengthened, the concept of this invention is useful).

Further, in MRI, when an appropriate contrast medium is developed, and high speed imaging is put to practical use and dynamic scanning is performed, the conception of the invention can be applied.

As described above in detail by the invention, a plurality of synchronised image of moving portions coincided in phase can be reconstructed, and in particular, a synchronised image reconstruction device can be provided which is most useful for dynamic study applications.

## Claims

1. A computer tomographic system for dynamic scanning of an object (10) which is subject to cyclic displacement while said object is exposed to radiation energy, comprising:

   scanning means (11, 12, 14) for generating consecutive projection data representing a measured characteristic of said object by scanning a portion of said object (10) over a selected time period;

   detecting means (13) for generating displacement signals responsive to the cyclic displacement of said object during said time period;

   memory means (14, 19) coupled to said scanning means and said detecting means for correlating with respect to time, and storing said consecutive projection data and said displacement signals;

   setting means (17) coupled to said memory means for setting specific phases of the displacement signals and reconstruction means (14) characterised in that the reconstruction means reads only one set of consecutive projection data from a region scanned within said object and corresponding displacement signals having said set specific phase to reconstruct an image of a section of said object and reconstructs a plurality of synchronized images in the time axis direction.

2. A computed tomographic system according to claim 1 wherein said setting means sets a derived phase range of projection data to be collected prior to and after the occurrence of a specified displacement signal.

3. A computed tomographic system according to claim 1 wherein said setting means sets a phase range starting at a first specified phase and ending at a second specified phase.

## Patentansprüche

1. Computertomographiesystem zur dynamischen Abtastung eines Objektes (10), das Gegenstand einer zyklischen Verlagerung ist, während das Objekt einer Strahlungsenergie ausgesetzt ist, mit:

   einer Abtasteinrichtung (11, 12, 14), um aufeinanderfolgende Projektionsdaten zu erzeugen, die eine gemessene Eigenschaft des Objektes darstellen, indem während einer vorbestimmten Zeitdauer ein Bereich des Objektes (10) abgetastet wird;

   einer Erfassungseinrichtung (13), um während der Zeitdauer Verlagerungssignale zu erzeugen, die der zyklischen Verlagerung des Objektes entsprechen;

   Speichereinrichtungen (14, 19), die mit der Abtasteinrichtung und der Erfassungseinrichtung gekoppelt sind, um in zeitlicher Hinsicht miteinander in Wechselbeziehung zu stehen, und in denen die aufeinanderfolgenden Projektionsdaten und die Verlagerungssignale gespeichert sind;

   einer mit den Speichereinrichtungen gekoppelten Einstelleinrichtung (17) zum Einstellen spezieller Phasen der Verlagerungssignale und einer Rekonstruktionseinrichtung (14), **dadurch gekennzeichnet**, daß durch die Rekonstruktionseinrichtung lediglich ein Satz aufeinanderfolgender Projektionsdaten aus einem Bereich, der in dem Objekt abgetastet wird, und zugehörige Verlagerungssignale, die die eingestellte spezielle Phase aufweisen, ausgelesen werden, um ein Bild eines Abschnittes des Objektes zu rekonstruieren, und eine Anzahl synchronisierter Bilder in Richtung der Zeitachse rekonstruiert wird.

2. Computertomographiesystem nach Anspruch 1, **dadurch gekennzeichnet**, daß durch die Einstelleinrichtung ein abgeleiteter Phasenbereich von Projektionsdaten eingestellt wird, um vor und nach dem Auftreten eines speziellen Verlagerungssignals erfaßt zu werden.

3. Computertomographiesystem nach Anspruch 1, **dadurch gekennzeichnet**, daß durch die Einstelleinrichtung ein Phasenbereich eingestellt wird, der bei einer ersten speziellen Phase beginnt und bei einer zweiten speziellen Phase endet.

## Revendications

1. Système de tomographie par ordinateur pour le balayage dynamique d'un objet (10) soumis à un déplacement périodique pendant que ledit objet est exposé à une énergie de rayonnement, comprenant :

   des moyens de balayage (11, 12, 14) pour générer des données de projection consécutives représentant la mesure d'une caractéristique dudit objet par balayage d'une partie dudit objet (10) pendant un intervalle de temps choisi ;

des moyens de détection (13) pour générer des signaux de déplacement correspondants au déplacement périodique dudit objet pendant ledit intervalle de temps ;

des moyens de mémoire (14, 19) couplés auxdits moyens de balayage et auxdits moyens de détection pour mémoriser lesdites données de projection consécutives et lesdits signaux de déplacement et les mettre en corrélation par rapport au temps ;

des moyens de réglage (17) couplés auxdits moyens de mémoire pour définir des phases spécifiques des signaux de déplacement et des moyens de reconstitution (14), caractérisé en ce que les moyens de reconstitution lisent seulement un ensemble de données de projection consécutives provenant d'une région balayée dans ledit objet et de signaux de déplacement correspondants ayant ladite phase spécifique définie pour reconstituer une image d'une partie dudit objet, et reconstituent une pluralité d'images synchronisées selon la direction de l'axe temporel.

2. Système de tomographie par ordinateur selon la revendication 1, dans lequel lesdits moyens de réglage définissent une gamme de phases dérivée des données de projection devant être collectées avant et après l'apparition d'un signal de déplacement spécifié.

3. Système de tomographie par ordinateur selon la revendication 1, dans lequel lesdits moyens de réglage définissent une gamme de phases commençant à une première phase spécifiée et finissant à une deuxième phase spécifiée.

FIG.1A.

FIG.1B.

FIG.2.

| SOURCE POSITION t = | PROJECTION NUMBER | PHASE OF WAVE 'R' $\emptyset =$ (+)   (–) | | E.C.G. 0 |
|---|---|---|---|---|
| 1 | 1 | 1 , –141 | | |
| 2 | (2) | 2 , –140 | | |
| 3 | (3) | 3 , –139 | | |
| ¦ | ¦ | ¦ ¦ | | |
| ¦ | ¦ | ¦ ¦ | | |
| 136 | (136) | 136 , –6 | | P |
| 137 | (137) | 137 , –5 | | |
| 138 | (138) | 138 , –4 | | |
| 139 | (139) | 139 , –3 | | |
| 140 | (140) | 140 , –2 | | Q |
| 141 | (141) | 141 , –1 | | |
| 142 | (142) | 0 , 0 | | R |
| 143 | (143) | 1 , –348 | | |
| 144 | (144) | 2 , –347 | | S |
| ¦ | ¦ | ¦ ¦ | | |
| ¦ | ¦ | ¦ ¦ | | |
| 490 | (490) | 348 , –1 | | |
| 491 | (491) | 0 , 0 | | R |
| 492 | (492) | 1 , –372 | | |
| 493 | (493) | 2 , –371 | | |
| 494 | (494) | 3 , –370 | | S |
| ¦ | ¦ | ¦ ¦ | | |
| ¦ | ¦ | ¦ ¦ | | |

$$F_{IG}.3.$$

FIG.4.

| SLICE NO | PROJECTION NO | PHASE $\phi$ OF WAVE "R" | |
|---|---|---|---|
| | | $\phi_1$ | $\phi_2$ |
| 1 | (2) ~ (282) | −140 ~ | 140 |
| 2 | (351) ~ (631) | −140 ~ | 140 |
| 3 | 722 ~ (1002) | −140 ~ | 140 |
| 4 | (991) ~ (1271) | −140 ~ | 140 |
| 5 | (1306) ~ (1586) | −140 ~ | 140 |
| 6 | (1674) ~ (1954) | −140 ~ | 140 |
| 15 | (4757) ~ (5037) | −140 ~ | 140 |

FIG. 5.

SLICE NO. 15
SLICE NO. 3
SLICE NO. 2
SLICE NO.1

FIG. 6.

DISPLAY

SLICE NO.1

15

ROI

17

DIALOGUE MACHINE

14

COMPUTER

20

POSITIONING OF ROI

FIG. 7.

11

FIG.8.

PHASE OF WAVE "R" TO "R"

| SLICE NO | PROJECTION NO | | |
|---|---|---|---|
| 1 | (142) | ~ | (490) |
| 2 | (491) | ~ | (771) |
| 3 | (862) | ~ | (1142) |
| 4 | (1131) | ~ | (1411) |
| 5 | (1446) | ~ | (1726) |
| 6 | (1814) | ~ | (2094) |
| 15 | (4897) | ~ | (5177) |

FIG. 10.

FIG. 9.

FIG. IIA.

FIG. IIB.

EP 0 257 922 B1

FIG.12.

EP 0 257 922 B1